(19) Europäisches Patentamt
European Patent Office
Office européen des brevets

(11) Veroffentlichungsnummer **0 004 368**
**B1**

(12) **EUROPÄISCHE PATENTSCHRIFT**

(45) Veroffentlichungstag der Patentschrift
**04.11.81**

(51) Int Cl.³ **A 61 K 7/13,** C 07 D 235/04

(21) Anmeldenummer **79100831.1**

(22) Anmeldetag **19.03.79**

(54) **Haarfärbemittel.**

(30) Prioritat **23.03.78 DE 2812678**

(43) Veroffentlichungstag der Anmeldung
03.10.79 **Patentblatt 79/20**

(45) Bekanntmachung des Hinweises auf die Patenterteilung
**04.11.81 Patentblatt 81/44**

(84) Benannte Vertragsstaaten
**BE CH FR GB IT NL SE**

(56) Entgegenhaltungen:
**DE-A-1 492 166**
**DE-A-1 921 911**

(73) Patentinhaber **Henkel Kommanditgesellschaft auf Aktien, -Patentabteilung-**
**Postfach 1100 Henkelstrasse 67,**
**D-4000 Düsseldorf 1 (DE)**

(72) Erfinder **Konrad, Günther, Dr., Feuerbachweg 12, D-4010 Hilden (DE)**
Erfinder: **Rose, David, Dr., Holbeinweg 7, D-4010 Hilden (DE)**
Erfinder **Lieske, Edgar, Hunsrückenstrasse 40, D-4000 Düsseldorf (DE)**

**EP 0 004 368 B1**

Anmerkung Innerhalb von neun Monaten nach der Bekanntmachung des Hinweises auf die Erteilung des europaischen Patents im Europäischen Patentblatt kann jedermann beim Europäischen Patentamt gegen das erteilte europaische Patent Einspruch einlegen Der Einspruch ist schriftlich einzureichen und zu begründen. Er gilt erst als eingelegt, wenn die Einspruchsgebühr entrichtet worden ist (Art. 99(1) Europaisches Patentübereinkommen)

Haarfärbemittel

Gegenstand der Erfindung sind Mittel zur oxidativen Färbung von Haaren auf Basis von Benzimidazolderivaten als Kupplerkomponente.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Aminopyrazolonderivate, heterocyclische Hydrazone eingesetzt. Als sogenannte Kupplerkomponenten werden Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein. Weiterhin ist von Bedeutung, daß auf dem zu färbenden Haar möglichst kräftige und den natürlichen Haarfarbennuancen weitgehend entsprechende Farbtöne erhalten werden. Ferner kommt der allgemeinen Stabilität der gebildeten Farbstoffe sowie deren Lichtechtheit, Waschechtheit und Thermostabilität ganz besondere Bedeutung zu, um Farbverschiebungen von der ursprünglichen Farbnuance oder gar Farbumschläge in andere Farbtöne zu vermeiden.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß Haarfärbemittel auf Basis von Oxidationsfarbstoffen mit einem Gehalt an Benzimidazolderivaten der allgemeinen Formel

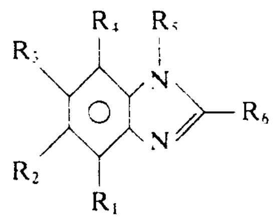

in der $R_1$ bis $R_4$ Wasserstoff, Hydroxygruppen bzw. Methoxygruppen darstellen, mit der Maßgabe, daß folgende Kombinationen gelten sollen:

1) $R_1 = R_4 = OH$, $R_2 = R_3 = H$
2) $R_1 = R_4 = OCH_3$, $R_2 = R_3 = H$
3) $R_1 = R_4 = H$, $R_2 = R_3 = OH$
4) $R_1 = R_4 = H$, $R_2 = R_3 = OCH_3$,

$R_5$ Wasserstoff oder ein Methylrest und $R_6$ Wasserstoff, ein Methylrest oder ein Phenylrest ist, sowie deren anorganischen oder organischen Salzen als Kupplersubstanzen und den in Oxidationshaarfarben üblichen Entwicklerkomponenten den gestellten Anforderungen in besonders hohem Maße gerecht werden.

Bei ihrem Einsatz als Kupplerkomponenten liefern die erfindungsgemäßen Verbindungen mit den im allgemeinen für die Oxidationshaarfärbung verwendeten Entwicklersubstanzen sehr intensive, von braun bis schwarzblau reichende Farbnuancen und stellen somit eine wesentliche Bereicherung der oxidativen Haarfärbemöglichkeiten dar. Darüber hinaus zeichnen sich die erfindungsgemäßen Benzimidazolderivate durch sehr gute Echtheitseigenschaften der damit erzielten besonders kräftigen Färbungen, durch eine gute Löslichkeit im Wasser, eine gute Lagerstabilität und toxikologische sowie dermatologische Unbedenklichkeit aus.

Die erfindungsgemäß als Kupplerkomponenten zu verwenderden Benzimidazolderivate können entweder als solche oder in Form ihrer Salze mit anorganischen oder organischen Säuren, wie z. B. als Chloride, Sulfate, Phosphate, Acetate, Propionate, Lactate, Citrate eingesetzt werden.

Die erfindungsgemäß als Kupplerkomponenten zu verwendenden Benzimidazolderivate stellen bekannte Verbindungen dar oder können nach allgemein bekannten Verfahren hergestellt werden, wie sie von L. Weinberger und A.R. Day im J. of Organic Chemistry 24 (1959) Seite 1453 beschrieben werden.

Als erfindungsgemäß zu verwendende Kupplerkomponenten sind 4,7-Dihydroxy-, 4,7-Dihydroxy-1-methyl-, 4,7-Dihydroxy-2-methyl-, 4,7-Dihydroxy-1,2-dimethyl-benzimidazol-hydrobromid, 4,7-Dimethoxy-, 4,7-Dimethoxy-1-methyl-, 4,7-Dimethoxy-2-methyl-benzimidazol, 5,6-Dihydroxy-, 5,6-Dihydroxy-1-methyl-, 5,6-Dihydroxy-2-methyl-, 5,6-Dihydroxy-2-phenyl-benzimidazol-hydrobromid,

5,6-Dimethoxy-, 5,6-Dimethoxy-1-methyl-, 5,6-Dimethoxy-2-methyl-, 5,6-Dimethoxy-2-phenyl-, 5,6-Dimethoxy-1,2-dimethyl-benzimidazol zu nennen.

Als Beispiele für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, Monochlor-p-phenylendiamin, p-Dimethylaminoanilin, p-Aminophenol, p-Diaminoanisol bzw. andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1–4 Kohlenstoffatomen darstellt, ferner Diaminopyridinderivate, heterocyclische Hydrazonderivate, 4-Aminopyrazolonderivate, wie 4-Amino-1-phenyl-3-carbamoylpyrazolon-5 anzuführen.

Als weitere Entwicklerkomponenten, die sich mit Vorteil mit den Benzimidazolderivaten kombinieren lassen, sind Tetraaminopyrimidine der allgemeinen Formel

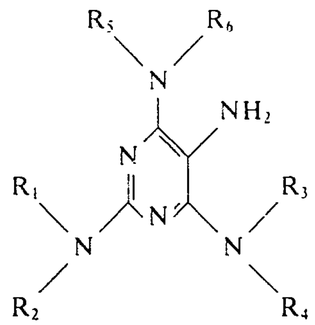

in der $R_1-R_6$ Wasserstoff, einen Alkylrest mit 1–4 Kohlenstoffatomen, den Rest $-(CH_2)_n-X$, in dem $n=1-4$ und X eine Hydroxylgruppe, ein Halogenatom, eine $-NH_2$-, $-NHR'$- und $-NR'R''$-Gruppe sein können, wobei R' und R'' Alkylreste mit 1–4 Kohlenstoffatomen bedeuten können oder mit dem Stickstoffatom zu einem heterocyclischen Ring, der ein weiteres Stickstoffatom oder Sauerstoffatom enthalten kann, geschlossen sind, $R_1$ und $R_2$, bzw. $R_3$ und $R_4$, bzw. $R_5$ und $R_6$ mit dem jeweiligen Stickstoffatom einen heterocyclischen 5- oder 6-gliedrigen Ring mit einem oder zwei Stickstoffatomen oder einem Stickstoffatom und einem Sauerstoffatom darstellen können sowie deren anorganische oder organische Salze zu nennen.

Die als Entwicklerkomponenten zu verwendenden Tetraaminopyrimidine können entweder als solche oder in Form ihrer Salze mit anorganischen oder organischen Säuren, wie z. B. als Chloride, Sulfate, Phosphate, Acetate, Propionate, Lactate, Citrate eingesetzt werden.

Für die Kombination mit den erfindungsgemäß als Kupplerkomponenten zu verwendenden Benzimidazolderivaten geeignete Entwicklersubstanzen stellen zum Beispiel

2,4,5,6-Tetraamino-,
4,5-Diamino-2,6-bismethylamino-,
2,5-Diamino-4,6-bismethylamino-,
4,5-Diamino-6-butylamino-2-dimethylamino-,
2,5-Diamino-4-diäthylamino-6-methylamino-,
4,5-Diamino-6-diäthylamino-2-dimethylamino-,
4,5-Diamino-2-diäthylamino-6-methylamino-,
4,5-Diamino-2-dimethylamino-6-äthylamino-,
4,5-Diamino-2-dimethylamino-6-isopropylamino-,
4,5-Diamino-2-dimethylamino-6-methylamino-,
4,5-Diamino-6-dimethylamino-2-methylamino-,
4,5-Diamino-2-dimethylamino-6-propylamino-,
2,4,5-Triamino-6-dimethylamino-,
4,5,6-Triamino-2-dimethylamino-,
2,4,5-Triamino-6-methylamino-,
4,5,6-Triamino-2-methylamino-,
4,5-Diamino-2-dimethylamino-6-piperidino-,
4,5-Diamino-6-methylamino-2-piperidino-,
2,4,5-Triamino-6-piperidino-,
2,4,5-Triamino-6-anilino-,
2,4,5-Triamino-6-benzylamino-,
2,4,5-Triamino-6-benzylidenamino-,
4,5,6-Triamino-2-piperidino-,
2,4,6-Trismethylamino-5-amino-,
2,4,5-Triamino-6-di-n-propylamino-,
2,4,5-Triamino-6-morpholino-,

2,5,6-Triamino-4-dimethylamino-,
4,5,6-Triamino-2-morpholino-,
2,4,5-Triamino-6-β-hydroxyäthylamino-,
4,5,6-Triamino-2-β-amino-äthylamino-,
2,5,6-Triamino-4-β-methylamino-äthylamino-,
2,5-Diamino-4,6-bis-γ-diäthylamino-propylamino-,
4,5-Diamino-2-methylamino-6-β-hydroxy-äthylamino-,
5-Amino-2,4,6-triäthylamino-,
2,4-Bis-β-hydroxyäthylamino-6-anilino-5-amino-pyrimidin

dar.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt. Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß einzusetzenden Benzimidazolderivate darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, d. h. die Entwicklung der Färbung, kann grundsätzlich, wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z. B. Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Äthylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholestrin zu nennen. Die genannten Zusatzstoffe werden dabei in den für diese Zwecke üblichen Mengen eingesetzt, wie z. B. Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40° C. Nach einer Einwirkungsdauer von circa 30 Minuten wird das Haarfärbemittel vom zu färbenden Haar durch Spülen entfernt. Hernach wird das Haar mit einem milden Shampoo nachgewaschen und getrocknet.

Aus der deutschen Offenlegungsschrift 1 921 911 sind bereits Haarfärbemittel bekannt, die als Kupplersubstanzen Benzimidazolderivate der Formel

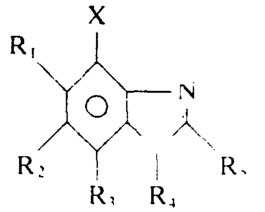

enthalten, in der X ein Hydroxylradial oder eine Aminogruppe bedeutet und $R_1$, $R_2$, $R_3$, $R_5$ Wasserstoff oder einen Methylrest darstellen, wobei mindestens einer der Reste $R_1$ und $R_3$ Wasserstoff und $R_4$ Wasserstoff oder ein Alkylrest mit 1–4 C-Atomen ist. Diese Formulierung »mindestens einer der Reste $R_1$ und $R_3$" besagt aber, daß besonders gute Färbeergebnisse erzielt werden, wenn beide Reste $R_1$ und $R_3$ Wasserstoff darstellen. Es war daher überraschend, daß eine weitere Substitution durch eine zweite Hydroxygruppe in p-Stellung ($R_3$) zur ersten Hydroxygruppe (X) bzw. eine gänzlich neue Substitution in Stellung $R_1$ und $R_2$ durch Hydroxygruppen zu besonders brauchbaren

Kupplersubstanzen führt.

Ferner sind aus der deutschen Offenlegungsschrift 1 492 166 Haarfärbemittel bekannt, die sich der Oxy- oder Amino-Indazolderivate als Kupplersubstanzen bedienen. In diesem Fall handelt es sich um ein gänzlich anderes Ringsystem des Grundkörpers, aus dem keine Rückschlüsse auf die erfindungsgemäß verwendeten Substanzen gezogen werden können, wobei insbesondere auch kein Anzeichen dafür vorliegt, daß eine bevorzugte Stellung der zwei Hydroxygruppen zueinander zu einer überraschend günstigen Eignung als Kupplersubstanz führt.

Die nachfolgenden Beispiele sollen den Erfindungsgegenstand näher erläutern, ohne ihn jedoch hierauf zu beschränken.

## Beispiele

Die in den nachfolgenden Beispielen als Kupplerkomponenten einzusetzenden Benzimidazolderivate wurden entsprechend den Angaben von L. Weinberger und A. R. Day in J. of Organic Chemistry 24 (1959) Seite 1453 hergestellt.

Als Entwicklerkomponenten wurden in den folgenden Beispielen die nachstehend genannten Verbindungen eingesetzt:

E 1 2,4,5,6-Tetraaminopyrimidin,
E 2 p-Toluylendiamin,
E 3 p-Phenylendiamin,
E 4 p-Aminophenol,
E 5 N,N-Dimethyl-p-phenylen-diamoniumchlorid,
E 6 2-Morpholino-4,5,6-triaminopyrimidin.

Als Kupplerkomponenten dienten folgende Verbindungen:

K 1 4,7-Dihydroxy-benzimidazol-hydrobromid,
K 2 5,6-Dihydroxy-benzimidazol-hydrobromid,
K 3 4,7-Dimethoxy-benzimidazol,
K 4 5,6-Dimethoxy-benzimidazol,
K 5 4,7-Dihydroxy-1-methyl-benzimidazol-hydrobromid,
K 6 5,6-Dihydroxy-1-methyl-benzimidazol-hydrobromid,
K 7 5,6-Dihydroxy-2-methyl-benzimidazol-hydrobromid,
K 8 5,6-Dihydroxy-2-phenyl-benzimidazol-hydrobromid.

Die erfindungsgemäßen Haarfärbemittel wurden in Form einer Cremeemulsion eingesetzt. Dabei wurden in eine Emulsion aus

10 Gew.-Teilen Fettalkoholen der Kettenlänge $C_{12}-C_{18}$,
10 Gew.-Teilen Fettalkoholsulfat (Natriumsalz) der Kettenlänge $C_{12}-C_{18}$,
75 Gew.-Teilen Wasser

jeweils 0,01 Mol der in der nachstehenden Tabelle aufgeführten Entwicklersubstanzen und Benzimidazolderivate eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde entweder mit Luftsauerstoff oder mit 1%iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit oder ohne zusätzlichem Oxidationsmittel wurde auf zu 90% ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | a) Entwickler | b) Kuppler | Erhaltener Farbton bei Luftoxidation | mit 1%iger $H_2O_2$-Lösung |
|---|---|---|---|---|
| 1 | E 1 | K 1 | graubraun | graubraun |
| 2 | E 2 | K 1 | blaugrau | schwarzblau |
| 3 | E 3 | K 1 | grau | rotgrau |
| 4 | E 4 | K 1 | graubraun | graubraun |
| 5 | E 5 | K 1 | grüngrau | grüngrau |
| 6 | E 6 | K 1 | graubraun | hellbraun |
| 7 | E 1 | K 2 | khaki | khaki |
| 8 | E 2 | K 2 | rotgrau | violettgrau |
| 9 | E 3 | K 2 | purpurgrau | violettgrau |
| 10 | E 4 | K 2 | graubraun | rotbraun |
| 11 | E 5 | K 2 | blaugrau | graublau |
| 12 | E 6 | K 2 | gelbbraun | gelbbraun |
| 13 | E 1 | K 3 | khaki | graugelb |
| 14 | E 2 | K 3 | nougatfarbig | graubraun |
| 15 | E 1 | K 4 | goldgelb | graugelb |
| 16 | E 2 | K 4 | grau | dunkelbraun |
| 17 | E 2 | K 5 | dunkelgrau | rotgrau |
| 18 | E 1 | K 6 | oliv | oliv |
| 19 | E 2 | K 6 | grüngrau | rauchfarbig |
| 20 | E 1 | K 7 | gelbbraun | braunorange |
| 21 | E 2 | K 7 | graubraun | biberbraun |
| 22 | E 1 | K 8 | gelbbraun | gelbbraun |
| 23 | E 2 | K 8 | olivbraun | olivbraun |

**Patentansprüche**

1. Haarfärbemittel auf Basis von Oxidationsfarbstoffen, gekennzeichnet durch einen Gehalt an Benzimidazolderivaten der allgemeinen Formel

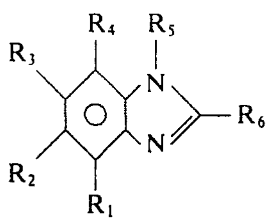

in der $R_1$ bis $R_4$ Wasserstoff, Hydroxygruppen bzw. Methoxygruppen darstellen, mit der Maßgabe, daß folgende Kombinationen gelten sollen:

1) $R_1 = R_4 = OH$, $R_2 = R_3 = H$
2) $R_1 = R_4 = OCH_3$, $R_2 = R_3 = H$
3) $R_1 = R_4 = H$, $R_2 = R_3 = OH$
4) $R_1 = R_4 = H$, $R_2 = R_3 = OCH_3$,

$R_5$ Wasserstoff oder ein Methylrest und $R_6$ Wasserstoff, ein Methylrest oder ein Phenylrest ist, sowie deren anorganischen oder organischen Salzen als Kupplersubstanzen und den in Oxidationsfarben üblichen Entwicklerkomponenten.

2. Haarfärbemittel nach Anspruch 1, gekennzeichnet durch einen Gehalt weiterer üblicher Kupplersubstanzen sowie gegebenenfalls üblicher direktziehender Farbstoffe.

## Claims

1. A hair-tinting agent based on oxidation dyes, characterised by a content of benzimidazole derivatives corresponding to the following general formula

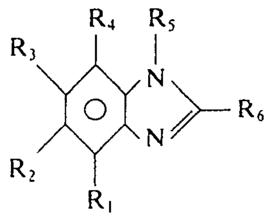

in which $R_1$ to $R_4$ represent hydrogen, hydroxy groups or methoxy groups with the proviso that the following combinations should apply:

| | |
|---|---|
| 1) $R_1 = R_4 = OH$, | $R_2 = R_3 = H$ |
| 2) $R_1 = R_4 = OCH_3$, | $R_2 = R_3 = H$ |
| 3) $R_1 = R_4 = H$, | $R_2 = R_3 = OH$ |
| 4) $R_1 = R_4 = H$, | $R_2 = R_3 = OCH_3$, |

$R_5$ represents hydrogen or a methyl radical and $R_6$ represents hydrogen, a methyl radical or a phenyl radical, and inorganic or organic salts thereof as couplers and the developer components normally used in oxidation dyes.

2. A hair-tinting agent as claimed in Claim 1, characterised by a content of other standard couplers and optionally standard substantive dyes.

## Revendications

1. Agents de teinture de cheveux à base de colorants d'oxydation, caractérisés par une teneur en dérivés de benzimidazol de formule générale:

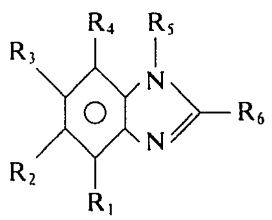

dans laquelle $R_1$ à $R_4$ représentent de l'hydrogène ou des groupes méthoxy, avec la mesure que les combinaisons suivantes doivent prévaloir:

| | |
|---|---|
| 1) $R_1 = R_4 = OH$, | $R_2 = R_3 = H$, |
| 2) $R_1 = R_4 = OCH_3$, | $R_2 = R_3 = H$, |
| 3) $R_1 = R_4 = H$, | $R_2 = R_3 = OH$, |
| 4) $R_1 = R_4 = H$, | $R_2 = R_3 = OCH_3$, |

$R_5$ est de l'hydrogène ou un radical méthyle et $R_6$ est de l'hydrogène, un radical méthyle ou un radical phényle, de même que leurs sels minéraux ou organiques, en tant que substances de copulation, et en composants de développement usuels dans les colorants d'oxydation.

2. Agents de teinture de cheveux selon la revendication 1, caractérisés par une teneur en d'autres substances usuelles de copulation de même qu'éventuellement en d'autres colorants usuels directement montants.